# EUROPEAN PATENT SPECIFICATION

(11) **EP 3 761 147 B1**
(45) Date of publication and mention of the grant of the patent: **22.11.2023**
(21) Application number: 19760690.8
(22) Date of filing: 27.02.2019
(51) Int. Cl.: G05D 21/02, G01D 21/02, A61M 16/10, F24F 11/30

(54) **DEVICE FOR MEASURING THE COMPOSITION OF MEDICAL AIR**
VORRICHTUNG ZUR MESSUNG DER ZUSAMMENSETZUNG VON MEDIZINISCHER LUFT
DISPOSITIF POUR MESURER LA COMPOSITION DE L'AIR MÉDICINAL

(30) Priority: 28.02.2018 ES 201830192
(43) Date of publication of application: 06.01.2021
(73) Proprietor: Ceimsa Electromedicina, S.L., 31600 Burlada (Navarra) (ES)
(72) Inventor: CRESPO MARTINEZ, Hermenegildo, 31600 Burlada (Navarra) (ES)
(74) Representative: Evens, Paul Jonathan
(86) International application number: PCT/ES2019/070117
(87) International publication number: WO 2019/166681

(56) References cited:
- WO-A1-2017/136336
- WO-A1-2017/180605
- CN-A- 106 943 653
- CN-A- 107 477 724
- CN-A- 107 741 723
- CN-U- 202 961 405
- US-A1- 2006 290 525
- US-A1- 2012 136 269
- Anonymous: "Analog Sensor Developer Kit", User Manual, Rev. 1.8, 1 November 2016 (2016-11-01), pages 1-4, XP055716344, Retrieved from the Internet: URL:https://www.spec-sensors.com/wp-conten t/uploads/2016/06/Analog-SDK-Users-Manual- v18.pdf [retrieved on 2020-07-20]

## Description

### Technical sector

The present invention is related to medical air supplied in health facilities or similar, and proposes a device that enables the composition of the medical air supplied to be known in real time.

### State of the art

In health facilities such as hospitals, clinics, health care centres or similar, it is common to have an installation of ducts through which medical air is supplied to operating rooms and other rooms where it is required. There are also other facilities in which it is highly important to know the air quality, such as fire department facilities, diving facilities, or facilities in the food sector, and in general any service that needs medical air supplied with certain guarantees.

Medical air has several different applications in the health sector, mainly being used to directly apply anaesthesia to the lungs by inhalation, or for ventilation therapies for patients with respiratory problems, who depend on a reliable supply of high quality medical air to protect their respiratory system, for which it is essential that the medical air is in the proper conditions when supplied.

Medical air can be supplied either by mixing oxygen and nitrogen, known as synthetic air by some companies, or produced by a compressor with adsorption dryers directly supplying the hospital network, or is bottled where it is compressed at a specific pressure and temperature. The medical air is formed by a composition of gases and oil mist that is controlled by regulations, these gases generally being 02, H2O, CO, CO2, NO, NO2 and SO2.

The bottles, mixing tanks, or compressors that incorporate the medical air are connected to centralised equipment that distributes the air to the rooms and operating rooms through the ducts of the facility, said rooms and operating rooms having equipment for supplying the air, which incorporate manometers for measuring the pressure of the air supplied. However, although this equipment allows the air pressure to be checked, it does not control the concentration of each element of the composition of the air to verify that it is within the range established by the regulations. For the case of synthetic air, the supplier carries out a control of the composition that is injected in the bottles in the factory, however, there is no final verification of the medical air obtained by purifying the atmospheric air. During storage, said composition may vary, or the air may become contaminated when it flows through the ducts of the health facility, resulting in air reaching patients that may not be within the standards established by the regulations, with the health risks this may entail. Fire department facilities and diving facilities also do not have verification equipment that continuously verifies the air, the majority of which is filled with compressors, and which certifies its breathable quality, and they do not have this equipment because, to date, there is no equipment that continuously measures all of the parameters.

It is therefore necessary to develop a solution that makes it possible to know in the very health facility the concentration of each element of the medical air composition that is being supplied to patients in compliance with the specification of the European Pharmacopoeia.

WO 2017/180605 relates to a breath analysis device, and discloses a device according to the pre-amble of appended independent claim 1.

US 2012/0136269 relates to liquid separation apparatus for removing a liquid from a respiratory gas and respiratory gas analyzing system.

### Summary of the invention

In accordance with the present invention, there is provided a device as defined in appended independent claim 1. Embodiments of the present invention are defined in appended claims dependent on independent claim 1. The invention relates to a device for measuring the composition of medical air in health facilities or similar, which enables the previously mentioned technical problem to be solved and also provides additional advantages that can be later used.

The device for measuring the composition of medical air comprises:
- an inlet branch that can be connected to a duct through which the medical air circulates;
- a sensor body in fluid communication with the inlet branch, for receiving a fraction of the medical air that circulates through the duct, and which has measuring modules, each module comprising a sensor for measuring the concentration of an element of the composition of the fraction of the medical air,
- an outlet branch in fluid communication with the sensor body, for carrying the fraction of the medical air to the outside of the device; and
- a control unit configured to receive the information measured by the sensors of the measuring modules.

Thus, a device is obtained that makes it possible to know in the very health facility the concentration of each element of the medical air composition that is being supplied to patients.

The inlet branch has a pressure regulator to adjust the pressure of the fraction of the medical air, a flow regulator to maintain a constant flow of the fraction of the medical air and a humidifier system to supply the fraction of the medical air to the sensor body in a dry or moist condition.

The sensor body may have an internal pressure sensor that determines the pressure in the sensor body, the control unit additionally being configured to act on the flow regulator based on said pressure and maintain a constant flow of the fraction of the medical air.

The humidifier system comprises a humidifier container and two solenoid valves that are configured to switch between a first state in which they allow the fraction of the medical air to pass through the humidifier container to supply the fraction of the medical air in a moist condition to the sensor body, and a second state in which the solenoid valves allow for the fraction of the medical air to directly pass to the sensor body in a dry condition.

Each measuring module has an internal memory with information on the calibration data and lifespan of the sensor, introduced in the process of initialising and adjusting the module, such that it is not necessary to calibrate the sensor each time it needs to be replaced, and furthermore, it is possible to know the end of the sensor's lifespan and thereby predict when it will fail.

Additionally, each measuring module has auxiliary power supply means to continuously supply electrical energy to the sensor of the measuring module, such that the sensor is always in the proper condition to take a reliable measurement.

Additionally, each measuring module may have low-noise amplifiers to adjust the measuring range of the sensor of the measuring module.

The measuring modules are independent units that have quick coupling means to connect to and disconnect from the sensor body, such that in the case of a failure of one of the sensors of the measuring modules, it is only necessary to disconnect the broken measuring module and connect a new one, and therefore there is no need to replace the entire device or unmount it from its position to do a repair.

The control unit is pneumatically isolated from the sensor body, but integral to the same and incorporates a microprocessor and control system that processes the data obtained by the measuring modules and calculates the concentration of each element of the composition of the analysed medical air using specific algorithms.

The device additionally may have display means to show the information received by the control unit.

The device may additionally comprise an external dew point sensor arranged in the duct through which the medical air flows, which measures the temperature of the dew point in the duct, the control unit additionally being configured to receive the datum of the temperature measured and to calculate a concentration of water vapour.

The device may additionally comprise an external pressure sensor arranged in the duct through which the medical air flows, which measures the pressure in the duct, the control unit additionally being configured to receive the datum of the pressure measured.

The device may additionally comprise an external flow sensor arranged in the duct through which the medical air flows, which measures the flow of the medical air in the duct, the control unit additionally being configured to receive the datum of the measured flow and to the consumption of medical air.

Thus, a measuring device is obtained that enables the composition of the medical air supplied to be known in real time and in the very health facility or similar, thereby preventing patients from receiving a medical composition that does not comply with regulations and, as such, would not be effective as a treatment or may even be harmful to the patient's health.

### Description of the figures

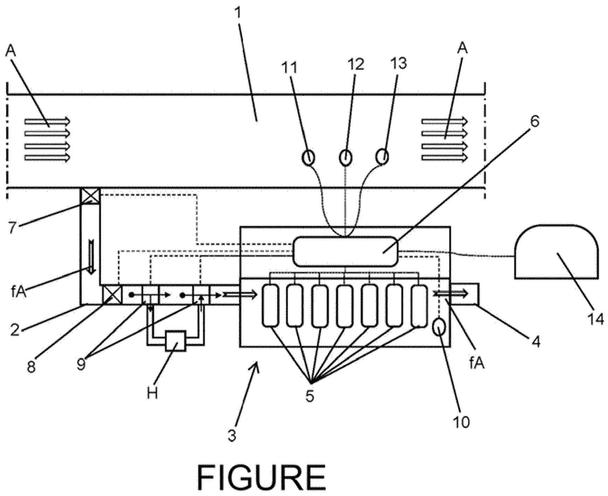

The figure shows a diagram of the device for measuring the composition of medical air of the invention.

### Detailed description of the invention

The invention relates to a device for measuring the composition of medical air (A) that flows through a duct (1) of a health facility such as a hospital, clinic, health care centre, nursing home, fire department facility, diving facility, facility in the food sector or any other similar building where the supply of medical air is required.

Generally, in health facilities, the duct (1) is connected at one end to a tank or set of bottles supplying medical air (A), and at the other end the duct (1) opens into the rooms of the health facility that require the medical air (A).

Device of the invention, on the fluid part thereof, comprises an inlet branch (2) that can be connected to a point of the duct (1) through which the medical air (A) circulates to obtain a fraction (fA)of the medical air (A), a sensor body (3) that analyses the fraction (fA) of the medical air (A) captured and an outlet branch (4) with a calibrated diameter that carries the analysed fraction (fA) of the medical air (A) out of the device.

The sensor body (3) has the shape of a box, which is sealed and in fluid communication with the inlet branch (2) to receive the fraction (fA) of the medical air (A). Likewise, the sensor body (3) contains measuring modules (5) therein, each module (5) incorporating a sensor to measure the concentration of an element of the composition of the fraction (fA) of the medical air (A).

In general, the elements measured in the composition of the fraction (fA) of the medical air (A) are oxygen (02), water (H2O), carbon monoxide (CO), carbon dioxide (CO2), nitrogen oxide (NO), nitrogen dioxide (NO2), sulfur dioxide (SO2) and oil mist, such that each sensor measures the concentration of an element of the composition, the device having as many measuring modules (5) as elements of the composition that are necessary to measure. Furthermore, inside the sensor body (3) the temperature of the medical air (fA) and the relative moisture thereof are measured.

The device has a control unit (6), pneumatically isolated from the sensor body (3) but mechanically integral to the same, which allows the device to be managed and is especially configured to receive the information measured by the sensors of the measuring modules (5) and to determine whether the composition of the medical air (A) that flows through the duct (1) is within regulations in force and, therefore, is the correct composition to be supplied. The control unit (6) also has an interface to connect to a data network.

The inlet branch (2) that connects the sensor body (3) to the duct (1) has a pressure regulator (7), a flow regulator (8) and a humidifier system (9,H). The pressure regulator (7) is arranged upstream from the inlet branch (2) according to the flow of the fraction (fA) of medical air (A) indicated by the arrows in the figure, the flow regulator (8) is arranged directly downstream from the pressure regulator (7), and the humidifier system (9, H) is arranged directly downstream from the flow regulator (8).

The pressure regulator (7) allows the pressure of the fraction (fA) of the medical air (A) that is supplied to the flow regulator (8) to be manually adjusted according to the optimal specification of its function.

The flow regulator (8) receives the fraction (fA) of the medical air (A) at the pressure established by the pressure regulator (7) and has the function of maintaining a constant flow of the fraction (fA) of the medical air (A) that will be measured in the sensor body (3). To do so, the sensor body (3) has an internal pressure sensor (10) that measures the pressure inside the sensor body (3), such that the pressure measurement is received by the control unit (6), which is additionally configured to command the flow regulator (8) based on said pressure and maintain a constant flow of the fraction (fA) of the medical air (A) that is supplied to the measuring modules (5) of the sensor body (3).

The flow regulator (8) preferably regulates the flow of the fraction (fA) of the medical air (A) between the values of 0.5 and 1.5 litres/minute.

The humidifier system (9, H) comprises two solenoid valves (9) and a humidifier container (H) with distilled water that allows medical air to be introduced in a dry or moist condition in the sensor body (3) based on the needs. The solenoid valves (9) are controlled by the control unit (6) which commands their opening and closing to alternate the passage of the fraction (fA) of the medical air (A) between a first state in which the fraction (fA) of the medical air (A) passes through the humidifier container (H) and a second state in which the fraction (fA) of the medical air (A) passes directly to the sensor body without being humidified. This way, the humidification of the sensors of the measuring modules (5) can be done at the desired frequency.

The measuring modules (5) are independent units that have conventional quick coupling means to connect to and disconnect from the sensor body (3), such as male-female pin connectors that allow for mechanical and electronic coupling, such that in the case of a failure or the end of the lifespan of a sensor, the entire measuring module (5) can be replaced in a relatively easy way without having to replace the entire device, but rather just the worn or damaged module.

Each measuring module (5) has an internal memory which has information related to the sensor of the measuring module (5). Said information comprises the installation date of the senor, calibration data of the sensor and the lifespan of the sensor. It is especially relevant to have the calibration data of the sensor, since it prevents having to calibrate the sensor every time the device is installed or a measuring module (5) is replaced. The information of the lifespan of the sensor is also relevant for being able to replace the measuring module (5) before a failure of the sensor occurs or before it begins to give erroneous measurements when at the end of its lifespan.

Each measuring module (5) has power supply means, such as a battery, that supplies continuous electrical energy to the sensor of the measuring module (5), and this way each sensor has an independent energy supply keeping it permanently operative. This way, the sensor is always prepared to take a reliable measurement when required to do so, since normally, given the time it takes the sensor to be electronically powered, it is necessary to wait a predefined time in order for it to provide reliable data, which in some cases can be up to 60 minutes, and therefore, having a measuring module (5) with continuous independent power for the sensor is optimal for the operation of the device.

Additionally, each measuring module (5) has low-noise amplifiers that allow the measuring range of the sensor of the measuring module (5) to be adjusted according to the needs.

The device additionally comprises a pressure sensor (11), a dew point sensor (12) and a flow sensor (13) that are arranged in the duct (1) through which the medical air (A) flows and which gather information on pressure, temperature of the dew point (to calculate the water content), and the flow of the medical air (A) in the duct (1). It is suitable to know this additional information of the duct (1) to complement the calculations with data obtained by the sensors of the measuring modules (5). Accordingly, the control unit (6) is additionally configured to receive the values measured by the sensors (11, 12, 13).

The device additionally comprises display means (14), such as a screen or display that shows all of the information measured by the device and received by the control unit (6), such that a visual control can be established of the composition of the medical air (A), the pressure, temperature and flow variables in the duct (1), and the relationship between the room temperature and the moisture outside the duct (1).

It is envisaged that the device of the invention be arranged in the duct (1) in an area near the point at which the medical air (A) supply bottles are connected, although this location is not limited, and it may be arranged at any other point in the duct (1) where there is a risk that the medical air supply could be contaminated.

## Claims

1. A device for measuring the composition of medical air (A), comprising:
• an inlet branch (2) that can be connected to a duct (1) through which the medical air (A) circulates;
• a sensor body (3) in fluid communication with the inlet branch (2), for receiving a fraction (fA) of the medical air (A) that circulates through the duct (1) and which has measuring modules (5), each module (5) comprising a sensor for measuring the concentration of an element of the composition of the fraction (fA) of the medical air (A);
• an outlet branch (4) in fluid communication with the sensor body (3), for carrying the fraction (fA) of the medical air (A) to the outside of the device; and
• a control unit (6) configured to receive the measurements and information of the sensors of the measuring modules (5),
wherein the inlet branch (2) has a pressure regulator (7) to adjust the pressure of the fraction (fA) of the medical air (A), and a flow regulator (8) to maintain a constant flow of the fraction (fA) of the medical air (A)
**characterized in that**:
the inlet branch (2) has a humidifier system (9,H) to supply the fraction (fA) of the medical air (A) to the sensor body (3) in a dry or moist condition, the humidifier system (9,H) comprising a humidifier container (H) and two solenoid valves (9) that are configured to switch between a first state in which they allow the fraction (fA) of the medical air (A) to pass through the humidifier container (H) to supply the fraction (fA) of the medical air (A) in a moist condition to the sensor body (3), and a second state in which they allow for the fraction (fA) of the medical air (A) to directly pass to the sensor body (3) in a dry condition, wherein the two solenoid valves (9) are controlled by the control unit (6); ;
each measuring module (5) has an internal memory with the information on the installation data, calibration data and lifespan of the sensor of the measuring module (5);
each measuring module (5) has power supply means for continuously supplying electrical energy to the sensor of the measuring module (5); and the measuring modules (5) are independent units that have quick coupling means to connect to and disconnect from the sensor body (3).

2. The device for measuring the composition of medical air (A), according to the preceding claim, wherein the sensor body (3) has an internal pressure sensor (10) that determines the pressure in the sensor body (3), the control unit (6) additionally being configured to act on the flow regulator (8) based on said pressure and maintain the constant flow of the fraction (fA) of the medical air (A).

3. The device for measuring the composition of medical air (A), according to any one of the preceding claims, wherein each measuring module (5) has low-noise amplifiers to adjust the measuring range of the sensor of the measuring module (5).

4. The device for measuring the composition of medical air (A), according to any one of the preceding claims, further comprising a pressure sensor (11) arranged in the duct (1) through which the medical air (A) flows, which measures the pressure in the duct (1), the control unit (6) being additionally configured to receive the measured pressure.

5. The device for measuring the composition of medical air (A), according to any one of the preceding claims, further comprising a dew point sensor (12) arranged in the duct (1) through which the medical air (A) flows, which measures the temperature of the dew point in the duct (1), the control unit (6) being additionally configured to receive the measured temperature and to calculate a concentration of water vapour.

6. The device for measuring the composition of medical air (A), according to any one of the preceding claims, further comprising a flow sensor (13) arranged in the duct (1) through which the medical air (A) flows, which measures the flow of the medical air (A) in the duct (1), the control unit (6) being additionally configured to receive the value of the flow and to calculate the consumption of medical air.

7. The device for measuring the composition of medical air (A), according to any one of the preceding claims, further comprising display means (14) for displaying the information received by the control unit (6).

## Patentansprüche

1. Vorrichtung zum Messen der Zusammensetzung von medizinischer Luft (A), umfassend:
• einen Einlasszweig (2), der mit einer Leitung (1) verbunden werden kann, durch den die medizinische Luft (A) zirkuliert;
• einen Sensorkörper (3) in Fluidverbindung mit dem Einlasszweig (2) zum Erhalten einer Fraktion (fA) der medizinischen Luft (A), die durch die Leitung (1) zirkuliert, und der Messmodule (5) aufweist, jedes Modul (5) umfassend einen Sensor zum Messen der Konzentration eines Elements der Zusammensetzung der Fraktion (fA) der medizinischen Luft (A);
• einen Auslasszweig (4) in Fluidverbindung mit dem Sensorkörper (3) steht, um die Fraktion (fA) der medizinischen Luft (A) nach außerhalb der Vorrichtung zu leiten; und
• eine Steuereinheit (6), die konfiguriert ist, um die Messungen und Informationen der Sensoren der Messmodule (5) zu erhalten,
wobei der Einlasszweig (2) einen Druckregler (7) zum Einstellen des Drucks der Fraktion (fA) der medizinischen Luft (A) und einen Strömungsregler (8) zum Aufrechterhalten einer konstanten Strömung der Fraktion (fA) der medizinischen Luft (A) aufweist
**dadurch gekennzeichnet, dass**:
der Einlasszweig (2) ein Befeuchtungssystem (9,H) aufweist, um dem Sensorkörper (3) die Fraktion (fA) der medizinischen Luft (A) in einem trockenen oder feuchten Zustand zuzuführen, das Befeuchtungssystem (9,H) umfassend einen Befeuchterbehälter (H) und zwei Magnetventile (9), die konfiguriert sind, um zwischen einem ersten Zustand, in dem sie die Fraktion (fA) der medizinischen Luft (A) durch den Befeuchterbehälter (H) hindurchlassen, um dem Sensorkörper (3) die Fraktion (fA) der medizinischen Luft (A) in einem feuchten Zustand zuzuführen, und einem zweiten Zustand, in dem sie die Fraktion (fA) der medizinischen Luft (A) in einem trockenen Zustand direkt zu dem Sensorkörper (3) durchlassen, wechseln, wobei die zwei Magnetventile (9) von der Steuereinheit (6) gesteuert werden;
jedes Messmodul (5) einen internen Speicher mit den Informationen über die Installationsdaten, Kalibrierungsdaten und betriebliche Lebensdauer des Sensors des Messmoduls (5) aufweist;
jedes Messmodul (5) Stromzuführungseinrichtungen aufweist, um dem Sensor des Messmoduls (5) kontinuierlich elektrische Energie zuzuführen; und die Messmodule (5) unabhängige Einheiten sind, die Schnellkupplungseinrichtungen aufweisen, um mit dem Sensorkörper (3) verbunden und davon getrennt zu werden.

2. Vorrichtung zum Messen der Zusammensetzung von medizinischer Luft (A) nach dem vorherigen Anspruch, wobei der Sensorkörper (3) einen internen Drucksensor (10) aufweist, der den Druck in dem Sensorkörper (3) bestimmt, wobei die Steuereinheit (6) zusätzlich konfiguriert ist, um basierend auf diesem Druck auf den Strömungsregler (8) einzuwirken und die konstante Strömung der Fraktion (fA) der medizinischen Luft (A) aufrechtzuerhalten.

3. Vorrichtung zum Messen der Zusammensetzung von medizinischer Luft (A) nach einem der vorherigen Ansprüche, wobei jedes Messmodul (5) rauscharme Verstärker aufweist, um den Messbereich des Sensors des Messmoduls (5) einzustellen.

4. Vorrichtung zum Messen der Zusammensetzung von medizinischer Luft (A) nach einem der vorherigen Ansprüche, ferner umfassend einen Drucksensor (11), der in der Leitung (1) angeordnet ist, durch die die medizinische Luft (A) strömt, der den Druck in der Leitung (1) misst, wobei die Steuereinheit (6) zusätzlich konfiguriert ist, um den gemessenen Druck zu erhalten.

5. Vorrichtung zum Messen der Zusammensetzung von medizinischer Luft (A) nach einem der vorherigen Ansprüche, ferner umfassend einen Taupunktsensor (12), der in der Leitung (1) angeordnet ist, durch die die medizinische Luft (A) strömt, der die Temperatur des Taupunkts in der Leitung (1) misst, wobei die Steuereinheit (6) zusätzlich konfiguriert ist, um die gemessene Temperatur zu erhalten und eine Konzentration von Wasserdampf zu berechnen.

6. Vorrichtung zum Messen der Zusammensetzung von medizinischer Luft (A) nach einem der vorherigen Ansprüche, ferner umfassend einen Strömungssensor (13), der in der Leitung (1) angeordnet ist, durch die die medizinische Luft (A) strömt, der die Strömung der medizinischen Luft (A) in der Leitung (1) misst, wobei die Steuereinheit (6) zusätzlich konfiguriert ist, um die den Wert der Strömung zu erhalten und den Verbrauch an medizinischer Luft zu berechnen.

7. Vorrichtung zum Messen der Zusammensetzung von medizinischer Luft (A) nach einem der vorherigen Ansprüche, ferner umfassend eine Anzeigeeinrichtung (14) zum Anzeigen der Informationen, die von der Steuereinheit (6) erhalten werden.

## Revendications

1. Dispositif de mesure de la composition d'un air médical (A), comprenant :
• une branche d'entrée (2) pouvant être reliée à un conduit (1) à travers lequel circule l'air médical (A) ;
• un corps de capteur (3) en communication fluidique avec la branche d'entrée (2), pour recevoir une fraction (fA) de l'air médical (A) qui circule à travers le conduit (1) et qui comporte des modules de mesure (5), chaque module (5) comprenant un capteur pour mesurer la concentration d'un élément de la composition de la fraction (fA) de l'air médical (A) ;
• une branche de sortie (4) en communication fluidique avec le corps de capteur (3), pour transporter la fraction (fA) de l'air médical (A) vers l'extérieur du dispositif ; et
• une unité de commande (6) configurée pour recevoir les mesures et les informations des capteurs des modules de mesure (5),
dans lequel la branche d'entrée (2) comporte un régulateur de pression (7) pour ajuster la pression de la fraction (fA) de l'air médical (A), et un régulateur de débit (8) pour maintenir un débit constant de la fraction (fA) de l'air médical (A)
**caractérisé en ce que** :
la branche d'entrée (2) comporte un système humidificateur (9, H) pour fournir la fraction (fA) de l'air médical (A) au corps de capteur (3) dans un état sec ou humide, le système humidificateur (9, H) comprenant un conteneur humidificateur (H) et deux électrovannes (9) qui sont configurées pour basculer entre un premier état dans lequel elles permettent à la fraction (fA) de l'air médical (A) de passer à travers le conteneur humidificateur (H) pour fournir la fraction (fA) de l'air médical (A) dans un état humide au corps de capteur (3), et un second état dans lequel elles permettent à la fraction (fA) de l'air médical (A) de passer directement vers le corps de capteur (3) dans un état sec, dans lequel les deux électrovannes (9) sont commandées par l'unité de commande (6) ;
chaque module de mesure (5) comporte une mémoire interne avec les informations sur les données d'installation, les données d'étalonnage et la durée de vie du capteur du module de mesure (5) ;
chaque module de mesure (5) comporte des moyens d'alimentation électrique pour fournir en continu de l'énergie électrique au capteur du module de mesure (5) ; et les modules de mesure (5) sont des unités indépendantes qui comportent des moyens de couplage rapide pour se connecter et se déconnecter du corps de capteur (3).

2. Dispositif de mesure de la composition de l'air médical (A), selon la revendication précédente, dans lequel le corps de capteur (3) comporte un capteur de pression interne (10) qui détermine la pression dans le corps de capteur (3), l'unité de commande (6) étant en outre configurée pour agir sur le régulateur de débit (8) en fonction de ladite pression et maintenir le débit constant de la fraction (fA) de l'air médical (A).

3. Dispositif de mesure de la composition de l'air médical (A), selon l'une quelconque des revendications précédentes, dans lequel chaque module de mesure (5) comporte des amplificateurs à faible bruit pour ajuster la plage de mesure du capteur du module de mesure (5).

4. Dispositif de mesure de la composition de l'air médical (A), selon l'une quelconque des revendications précédentes, comprenant en outre un capteur de pression (11) disposé dans le conduit (1) à travers lequel circule l'air médical (A), qui mesure la pression dans le conduit (1), l'unité de commande (6) étant en outre configurée pour recevoir la pression mesurée.

5. Dispositif de mesure de la composition de l'air médical (A), selon l'une quelconque des revendications précédentes, comprenant en outre un capteur de point de rosée (12) disposé dans le conduit (1) à travers lequel circule l'air médical (A), qui mesure la température du point de rosée dans le conduit (1), l'unité de commande (6) étant en outre configurée pour recevoir la température mesurée et pour calculer une concentration de vapeur d'eau.

6. Dispositif de mesure de la composition de l'air médical (A), selon l'une quelconque des revendications précédentes, comprenant en outre un capteur de débit (13) disposé dans le conduit (1) à travers lequel circule l'air médical (A), qui mesure la débit de l'air médical (A) dans le conduit (1), l'unité de commande (6) étant en outre configurée pour recevoir la valeur du débit et calculer la consommation d'air médical.

7. Dispositif de mesure de la composition de l'air médical (A), selon l'une quelconque des revendications précédentes, comprenant en outre des moyens d'affichage (14) pour afficher les informations reçues par l'unité de commande (6).
